# EUROPEAN PATENT APPLICATION

(11) **EP 4 725 938 A1**
(43) Date of publication of application: **15.04.2026**
(21) Application number: 24818640.5
(22) Date of filing: 04.06.2024
(51) Int. Cl.: C07C 271/20, C07C 271/22, C07C 229/16, A61K 31/7088, A61K 31/713, A61K 47/18, A61K 9/127

(54) **IONIZABLE LIPID COMPOUND, LIPID CARRIER COMPRISING SAME AND USE THEREOF**

(30) Priority: 07.06.2023 WO PCT/CN2023/098958
(71) Applicant: InnoVec Biotherapeutics, Beijing 100094 (CN)
(72) Inventor: PAN, Xinghua, Beijing 100094 (CN); WANG, Cheng, Beijing 100094 (CN)
(74) Representative: E. Blum & Co. AG
(86) International application number: PCT/CN2024/097253
(87) International publication number: WO 2024/251108

(57) **Abstract**

The present invention relates to an ionizable lipid compound, a lipid carrier comprising same and the use thereof. Provided in the present invention are a series of compounds as represented by formula (1), a lipid carrier comprising same as an ionizable lipid, a nucleic acid lipid nanoparticle composition and a preparation thereof. A lipid nanoparticle formed from the ionizable lipid can deliver a nucleic acid molecule into the body, so that the transfer rate of a nucleic acid molecule is increased, and the treatment effect of a nucleic acid drug is improved.

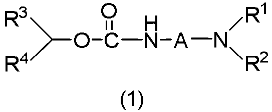

## Description

### TECHNICAL FIELD

The present disclosure belongs to the field of biological medicine, and specifically relates to an ionizable lipid compound and a lipid carrier comprising the same, a nucleic acid lipid nanoparticle composition, and a pharmaceutical preparation.

### BACKGROUND

Lipid nanoparticles are widely used in the field of drug delivery. Of these, ionizable lipids are not only excellent carriers for protein/polypeptide antigens, but also a novel type of immune adjuvant, which can directly activate antigen-presenting cells and enhance vaccine-induced immune responses. Therefore, ionizable lipids are widely used in the field of vaccines to encapsulate and transport nucleic acid molecules. Ionizable lipid is the most crucial component for enabling the targeting capability and delivery performance of lipid nanoparticles. It binds to negatively charged nucleic acids, facilitates endosomal escape and *in vivo* transfection of nucleic acid molecules, and exhibits many characteristics such as pH sensitivity. Ionizable lipids determine the delivery efficiency and transfection efficiency of the lipid nanoparticle delivery system. Therefore, an ionizable lipid designed with good targeting capability and delivery performance is a key element indispensable for lipid nanoparticles.

DLin-MC3-DMA (hereinafter referred to as MC3), as the most commonly-used novel ionizable lipid on the market at present, boasts the advantage of "low toxicity and high efficiency" and is the first ionizable lipid applied globally to siRNA liposome products (Onpattro). With the launch of Onpattro in the United States, MC3 and novel ionizable lipids have attracted more attention from scientists. Compared with common ionizable lipids such as DOTAP, DOTMA and DC-CHOL, such novel ionizable lipids as MC3 have lower toxicity and significantly improved drug loading capacity and safety.

There are currently four siRNA and mRNA drugs worldwide (including those under Emergency Use Authorization) that have been authorized by FDA, in all of which novel ionizable lipids are employed. Therefore, novel ionizable lipids holds broad application prospects in nucleic acid drug delivery.

### SUMMARY

The present disclosure provides a series of compounds represented by formula (1), a lipid carrier comprising the same as an ionizable lipid, a nucleic acid lipid nanoparticle composition, and a preparation thereof. The lipid nanoparticle formed of this ionizable lipid is capable of delivering nucleic acid molecules into the body, improving the transport efficiency of the nucleic acid molecules, and thus improving the therapeutic effect of the nucleic acid molecules.

In the first aspect, the present disclosure provides a compound represented by formula (1) or a pharmaceutically acceptable salt thereof: 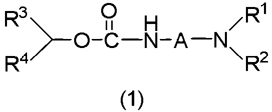 wherein
R¹ and R² are independently C₁₋₈ alkyl, and said alkyl is optionally substituted with one or more of OH, NH₂ or halogen;
A is C₁₋₈ alkylene;
R³ and R⁴ are independently -R⁶, -G³-O-C(=O)-R⁵, -G³-C(=O)-O-R⁵, -G³-NH-C(=O)-O-R⁵, -G³-NH-O-C(=O)-R⁵, -G³-NH-C(=O)-R⁵ or -G³-O-C(=O)-NH-R⁵;
G³ is independently C₂₋₁₀ alkylene;
R⁵ is independently C₂₋₃₀ branched alkyl or C₂₋₃₀ branched alkenyl, and said branched alkyl or branched alkenyl is optionally substituted with one or more of -O-C₁₋₃₀ alkyl, -C(=O)-O-C₁₋₃₀ alkyl, -O-C(=O)-C₁₋₃₀ alkyl, -O-C₂₋₃₀ alkenyl, -C(=O)-O-C₂₋₃₀ alkenyl or -O-C(=O)-C₂₋₃₀ alkenyl; and
R⁶ is C₂₋₃₀ alkenyl.

In some embodiments, R¹ and R² are independently C₁₋₆ alkyl, and said alkyl is optionally substituted with one or more of OH, NH₂ or halogen.

In some embodiments, R¹ and R² are independently C₁₋₄ alkyl, and said alkyl is optionally substituted with one or more of OH, NH₂ or halogen.

In some embodiments, R¹ and R² are independently -CH₃, -CH₂CH₃, -CH₂CH₂OH or - CH₂CH₂CH₃; or R¹ and R² are independently -CH₂CH₃ or -CH₂CH₂OH.

In some embodiments, A is C₁₋₆ alkylene.

In some embodiments, A is C₁₋₄ alkylene.

In some embodiments, A is C₂₋₄ alkylene.

In some embodiments, A is -CH₂-, -CH₂CH₂- or -CH₂CH₂CH₂-; or A is -CH₂CH₂-.

In some embodiments, R³ and R⁴ are independently -R⁶, -G³-O-C(=O)-R⁵, -G³-C(=O)-OR⁵, -G³-NH-C(=O)-O-R⁵ or -G³-NH-O-C(=O)-R⁵.

In some embodiments, R³ and R⁴ are independently -R⁶, -G³-O-C(=O)-R⁵ or -G³-C(=O)-OR⁵, or R³ and R⁴ are independently -R⁶ or -G³-O-C(=O)-R⁵; or R³ and R⁴ are independently -G³-O-C(=O)-R⁵.

In some embodiments, G³ is independently C₂₋₈ alkylene.

In some embodiments, G³ is independently C₂₋₆ alkylene.

In some embodiments, G³ is independently 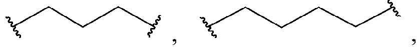 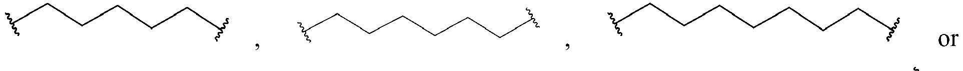 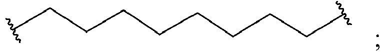 or G³ is independently

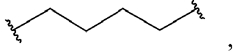

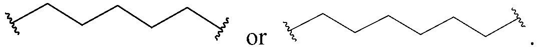

In some embodiments, R⁵ is C₂₋₂₄ branched alkyl or C₂₋₂₄ branched alkenyl, and said branched alkyl or branched alkenyl is optionally substituted with one or more of -O-C₁₋₂₄ alkyl, -C(=O)-O-C₁₋₂₄ alkyl, -O-C(=O)-C₁₋₂₄ alkyl, -O-C₂₋₂₄ alkenyl, -C(=O)-O-C₂₋₂₄ alkenyl or -O-C(=O)-C₂₋₂₄ alkenyl.

In some embodiments, R⁵ is C₄₋₂₄ branched alkyl or C₄₋₂₄ branched alkenyl, and said branched alkyl or branched alkenyl is optionally substituted with one or more of -O-C₁₋₂₄ alkyl, -C(=O)-O-C₁₋₂₄ alkyl, -O-C(=O)-C₁₋₂₄ alkyl, -O-C₂₋₂₄ alkenyl, -C(=O)-O-C₂₋₂₄ alkenyl or -O-C(=O)-C₂₋₂₄ alkenyl.

In some embodiments, R⁵ is C₆₋₂₄ branched alkyl or C₆₋₂₄ branched alkenyl, and said branched alkyl or branched alkenyl is optionally substituted with one or more of -O-C₁₋₂₄ alkyl, -C(=O)-O-C₁₋₂₄ alkyl, -O-C(=O)-C₁₋₂₄ alkyl, -O-C₂₋₂₄ alkenyl, -C(=O)-O-C₂₋₂₄ alkenyl or -O-C(=O)-C₂₋₂₄ alkenyl.

In some embodiments, R⁵ is C₈₋₂₄ branched alkyl or C₈₋₂₄ branched alkenyl, and said branched alkyl or branched alkenyl is optionally substituted with one or more of -O-C₁₋₂₄ alkyl, -C(=O)-O-C₁₋₂₄ alkyl, -O-C(=O)-C₁₋₂₄ alkyl, -O-C₂₋₂₄ alkenyl, -C(=O)-O-C₂₋₂₄ alkenyl or -O-C(=O)-C₂₋₂₄ alkenyl.

In some embodiments, R⁵ is C₁₀₋₂₀ branched alkyl or C₁₀₋₂₀ branched alkenyl, and said branched alkyl or branched alkenyl is optionally substituted with one or more of -O-C₁₋₂₀ alkyl, -C(=O)-O-C₁₋₂₀ alkyl, -O-C(=O)-C₁₋₂₀ alkyl, -O-C₂₋₂₀ alkenyl, -C(=O)-O-C₂₋₂₀ alkenyl or -O-C(=O)-C₂₋₂₀ alkenyl.

In some embodiments, R⁵ is

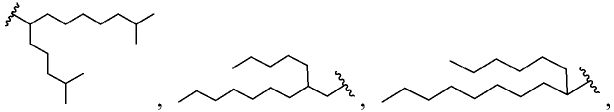

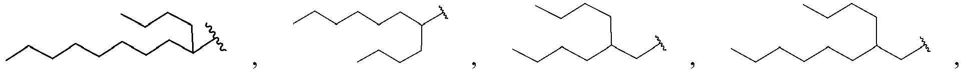

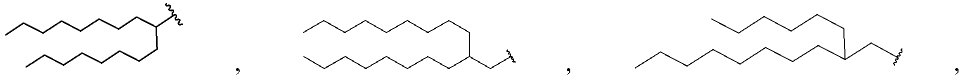

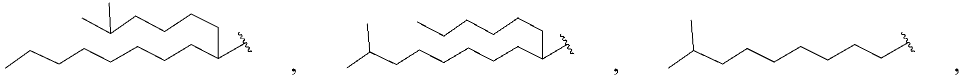

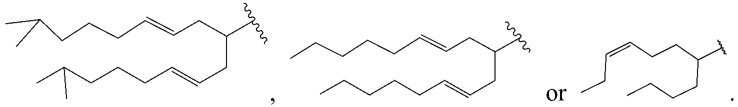

In some embodiments, R⁵ is 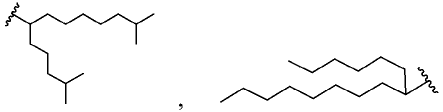 or

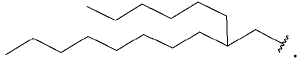

In some embodiments, R⁵ is

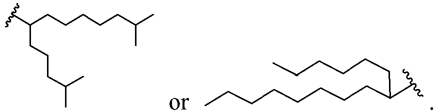

In some embodiments, R⁶ is C₂₋₂₄ alkenyl; or R⁶ is C₆₋₂₄ alkenyl; or R⁶ is C₈₋₂₄ alkenyl; or R⁶ is C₁₀₋₂₀ alkenyl.

In some embodiments, R⁶ is 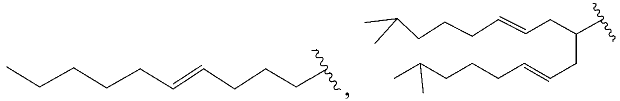 or

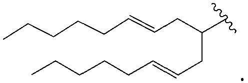

In some embodiments, R³ and R⁴ are independently 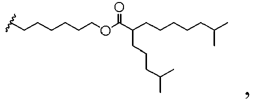 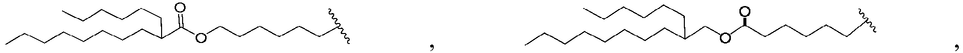 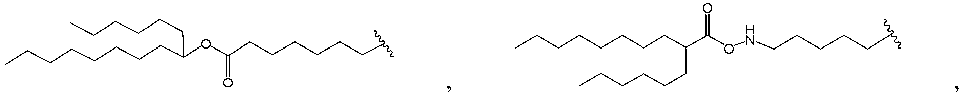 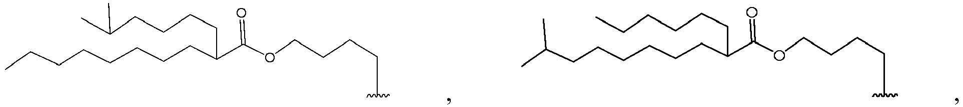 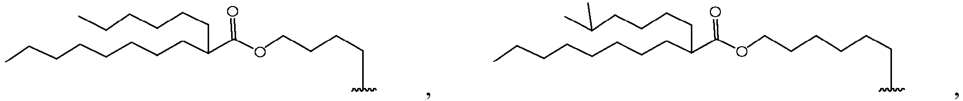 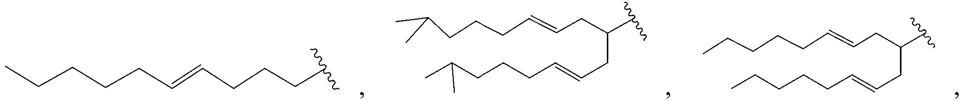 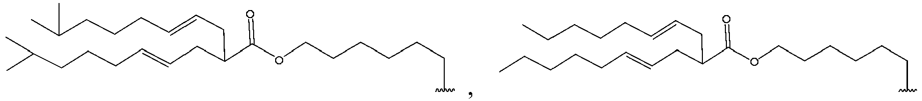 or

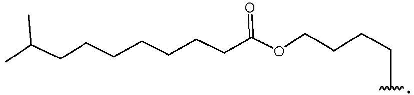

In some embodiments, R³ and R⁴ are independently

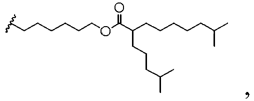

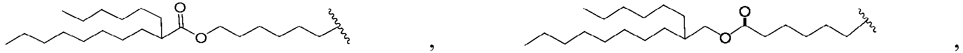

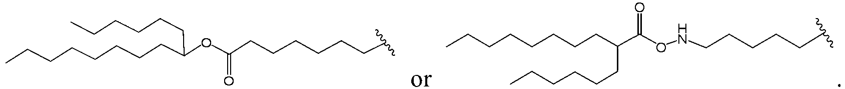

In some embodiments, R³ and R⁴ are independently 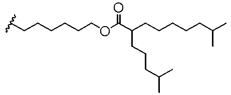 or

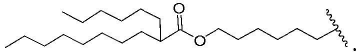

The present disclosure further provides a compound shown below or a pharmaceutically acceptable salt thereof:

| | Structure of Compound |
|---|---|
| 1 | 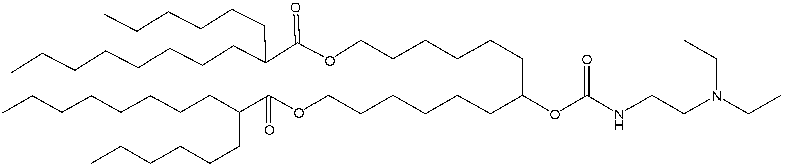 |
| 2 | 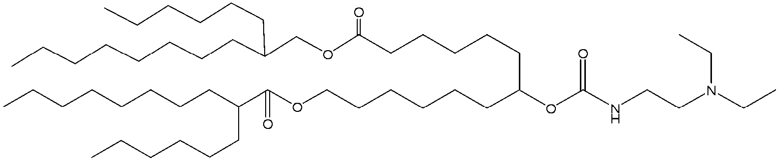 |
| 3 | 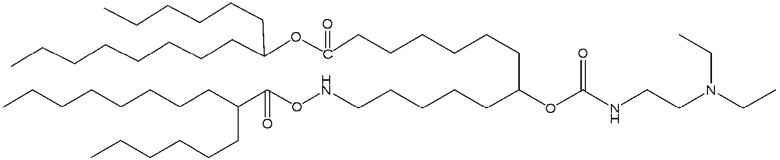 |
| 4 | 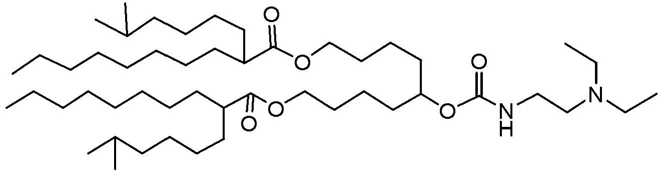 |
| 5 | 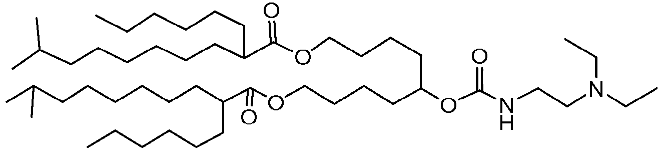 |
| 6 | 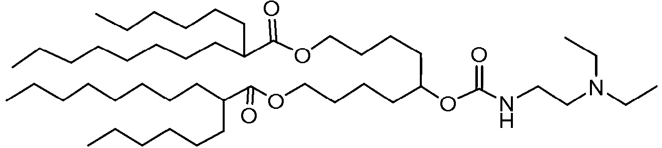 |
| 7 | 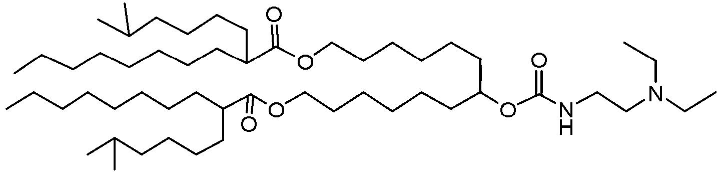 |
| 8 | 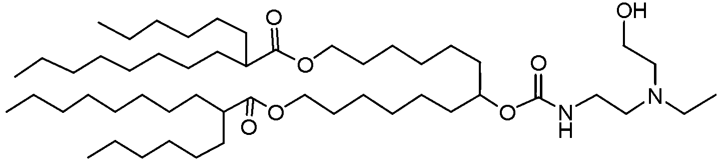 |
| 9 | 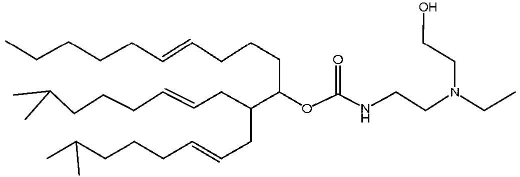 |
| 10 | 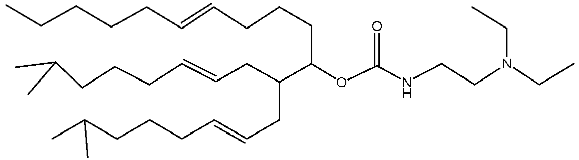 |
| 11 | 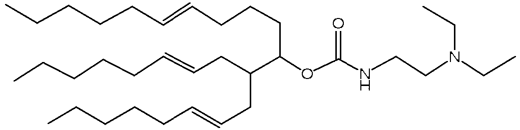 |
| 12 | 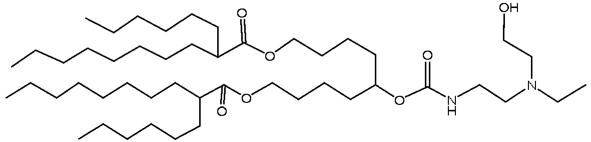 |
| 13 | 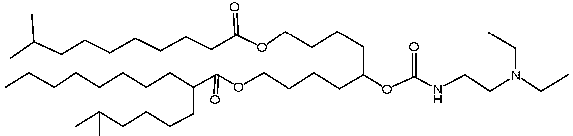 |
| 14 | 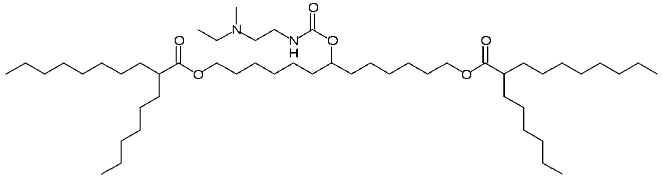 |
| 15 | 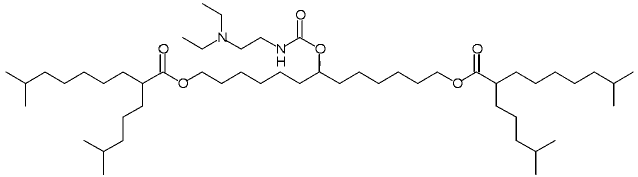 |

In the second aspect, the present disclosure provides a lipid carrier, comprising a compound represented by formula (1) or a pharmaceutically acceptable salt thereof as an ionizable lipid.

In some embodiments, the lipid carrier comprises a compound represented by formula (1) or a pharmaceutically acceptable salt thereof, a helper lipid, a structural lipid, and a polymer-conjugated lipid.

In some embodiments, the helper lipid is at least one selected from 1,2-dioleoyl-sn-glycero-3-phosphatidylethanolamine DOPE, 1,2-distearoyl-sn-glycero-3-phosphatidylcholine DSPC, dioleoylphosphatidylserine DOPS, distearoylphosphatidylserine DSPS, 1,2-distearoyl-sn-glycero-3-phosphoethanolamine DSPE, dipalmitoylphosphatidylserine DPPS, 1,2-dipalmitoyl-sn-glycero-3-phosphatidylcholine DPPC, 1,2-dioleoyl-sn-glycero-3-phosphatidylcholine DOPC, dipalmitoylphosphatidylglycerol DPPG, oleoylphosphatidylcholine POPC, 1-palmitoyl-2-oleoylphosphatidylethanolamine POPE, 1,2-dipalmitoyl-sn-glycero-3-phosphoethanolamine DPPE, 1,2-dimyristoyl-sn-glycero-3-phosphoethanolamine DMPE, distearoyl phosphatidylethanolamine DSPE, and 1-stearoyl-2-oleoylphosphatidylethanolamine SOPE.

In some embodiments, the helper lipid is at least one selected from 1,2-dioleoyl-sn-glycero-3-phosphatidylethanolamine DOPE and 1,2-distearoyl-sn-glycero-3-phosphatidylcholine DSPC.

In some embodiments, the structural lipid is at least one selected from cholesterol, nonsterol, sitosterol, ergosterol, campesterol, stigmasterol, brassicasterol, tomatine, ursolic acid, α-tocopherol, coprosterol, and corticosteroid.

In some embodiments, the structural lipid is cholesterol.

In some embodiments, the polymer-conjugated lipid is at least one selected from 1,2-dimyristoyl-rac-glycero-3-methoxypolyethylene glycol 2000 (DMG-PEG2000), DMG-PEG2000-mannose, cholesterol-PEG2000, 1,2-dimyristoyl-sn-glycerol methoxy-polyethylene glycol PEG-DMG, dimyristoylglycerol-polyethylene glycol PEG-c-DMG, polyethylene glycol-dimyristoylglycerol PEG-C14, PEG-1,2-dimyristoyloxypropyl-3-amine PEG-c-DMA, 1,2-distearoyl-sn-glycero-3-phosphoethanolamine-N-[amino(polyethylene glycol)] PEG-DSPE, PEGylated phosphatidylethanolamine PEG-PE, PEG-modified ceramide, PEG-modified dialkylamine, PEG-modified diacylglycerol, Tween-20, Tween-80, 1,2-dipalmityl-sn-glycero-methoxypolyethylene glycol PEG-DPG, 4-O-(2',3'-di(tetradecanoyloxy)propyl-1-O-(ω-methoxy(polyethoxy)ethyl)succinate PEG-s-DMG, PEG-dialkoxypropyl PEG-DAA, mPEG2000-1,2-di-O-alkyl-sn3-carbamoylglyceride PEG-c-DOMG, and N-acetylgalactosamine((R)-2,3-bis(octadecyloxy)propyl-1-(methoxypoly(ethylene glycol)2000)propylcarbamate)) GalNAc-PEG-DSG.

In some embodiments, the polymer-conjugated lipid is 1,2-dimyristoyl-rac-glycero-3-methoxypolyethylene glycol 2000 (DMG-PEG2000).

In some embodiments, in the lipid carrier, the molar ratio of the ionizable lipid, the helper lipid, the structural lipid, and the polymer-conjugated lipid is (20 to 75):(2 to 25):(15 to 55):(0 to 15); exemplarily, the molar ratio of the ionizable lipid, the helper lipid, the structural lipid and the polymer-conjugated lipid may be 45:10:42:3, 30:25:30:10, 46:15:40:3, 50:10:38.5:1.5, 50:10:37:3, 50:9:38:3, 60:5:34.5:0.5, 75:5:19.5:0.5, 65:5:29.5:0.5, 55:8:36.5:0.5, 50:8:41.5:0.5, 50:10:39.5:0.5, 50:9.5:40:0.5, etc.

In the third aspect, the present disclosure provides a nucleic acid lipid nanoparticle composition, comprising a compound represented by formula (1) or a pharmaceutically acceptable salt thereof or the aforesaid lipid carrier, and a nucleic acid.

In some embodiments, the nucleic acid is at least one selected from DNA, RNA, a complex containing DNA or RNA (*e.g.,* a complex of DNA and RNA, a complex of DNA and polypeptide/protein, and a complex of RNA and polypeptide/protein), a modified DNA, a modified RNA, and a modified complex containing DNA or RNA.

In some embodiments, the nucleic acid is RNA.

In some embodiments, the RNA is mRNA, siRNA, dsRNA, rRNA, circRNA, saRNA, tRNA, snRNA or shRNA, preferably mRNA.

In some embodiments, the mass ratio of the aforesaid nucleic acid to any one of the aforesaid compounds or pharmaceutically acceptable salt thereof is 1:(3 to 40).

In some embodiments, the mass ratio of the aforesaid nucleic acid to the aforesaid lipid carrier is 1:(3 to 40).

Exemplarily, the above mass ratio is 1:3, 1:5, 1:10, 1:15, 1:20, 1:30, etc.

In the fourth aspect, the present disclosure provides a pharmaceutical preparation, comprising any one of the aforesaid compounds or pharmaceutically acceptable salts thereof, or the aforesaid lipid carrier, or the aforesaid nucleic acid lipid nanoparticle composition, and a pharmaceutically acceptable carrier.

In some embodiments, the pharmaceutical preparation has a particle size of 30 to 500 nm; exemplarily, the particle size may be 30 nm, 40 nm, 50 nm, 60 nm, 70 nm, 80 nm, 90 nm, 100 nm, 110 nm, 120 nm, 130 nm, 140 nm, 150 nm, 200 nm, 250 nm, 300 nm, 350 nm, 500 nm, etc.

In some embodiments, the encapsulation efficiency of nucleic acids in the pharmaceutical preparation is greater than 50%. Exemplarily, the encapsulation efficiency may be 55%, 60%, 65%, 70%, 75%, 79%, 80%, 81%, 82%, 83%, 84%, 85%, 86%, 87%, 88%, 89%, 90%, 91%, 92%, 93%, 94%, 95%, 96%, 97%, 98%, 99%, etc.

In the fifth aspect, the present disclosure also provides use of the aforesaid compound or pharmaceutically acceptable salt thereof, or the aforesaid lipid carrier, or the aforesaid nucleic acid lipid nanoparticle composition, or the aforesaid pharmaceutical preparation in preparation of a nucleic acid drug or a gene vaccine.

The present disclosure further provides a method for *in vivo* delivery of a nucleic acid drug or a gene vaccine, the method comprising administering, to a subject in need thereof, the aforesaid nucleic acid lipid nanoparticle composition or the aforesaid pharmaceutical preparation.

The present disclosure further provides a method for treating or preventing a hepatic disease, the method comprising administering, to a subject in need thereof, the aforesaid nucleic acid lipid nanoparticle composition or the aforesaid pharmaceutical preparation, wherein the hepatic disease is hepatitis, fatty liver, liver fibrosis, cirrhosis, alcoholic/non-alcoholic liver injury or hepatocellular carcinoma.

The present disclosure further provides use of the aforesaid compound or pharmaceutically acceptable salt thereof or the aforesaid lipid carrier or the aforesaid nucleic acid lipid nanoparticle composition in preparation of a medicament for treating or preventing a hepatic disease.

In some embodiments, the aforesaid nucleic acid lipid nanoparticle composition or the aforesaid pharmaceutical preparation is administered via one of the following routes of administration: oral, intranasal, intravenous, intraperitoneal, intramuscular, intraarticular, intralesional, intratracheal, subcutaneous, and intradermal. In some embodiments, the aforesaid nucleic acid lipid nanoparticle composition or the aforesaid pharmaceutical preparation is administered, for example, via enteral or parenteral administration. In some embodiments, the nucleic acid lipid nanoparticle composition or the pharmaceutical preparation is administered to the subject at a dose of about 0.001 mg/kg to about 10 mg/kg.

### BRIEF DESCRIPTION OF THE DRAWINGS

FIG. 1: Proton nuclear magnetic resonance spectrum of compound 1.
FIG. 2: Chemiluminescence imaging of mice under experimental conditions 1, 2, 3, 4 and 5 in Example 5.
FIG. 3: Proton nuclear magnetic resonance spectrum of compound 14.
FIG. 4: Proton nuclear magnetic resonance spectrum of compound 15.

### DETAILED DESCRIPTION

To render the present disclosure more understandable, some technical and scientific terms are specifically defined below. Unless otherwise explicitly defined herein, all of the other technical and scientific terms used herein have the same meanings as typically understood by one of ordinary skill in the art to which the present disclosure belongs.

In the present specification, the numerical range represented by "numerical value A to numerical value B" refers to the range including the endpoint values A and B. When the upper limit and lower limit of a numerical range are disclosed, any value or any sub-range within this range means to be specifically disclosed. In particular, each of the numerical ranges (*e*.*g*., in the form of "from about a to b", or equivalently "from approximately a to b", or equivalently "about a-b") of the parameters disclosed herein should be construed to encompass each of the values and sub-ranges therein. For example, "C₁₋₄" should be construed to encompass any sub-range and each point value therein, *e.g.,* C₂₋₄, C₃₋₄, C₁₋₂, C₁₋₃ or C₁₋₄, and C₁, C₂, C₃ or C₄.

The term "including", "comprising", "having" or "containing" or any other variations thereof is intended to encompass non-exclusive or open-ended inclusions. For example, a composition, method or apparatus including a series of elements is not necessarily limited only to the elements that have been explicitly listed, and may also include other elements that are not explicitly listed or elements innate in the above composition, method or apparatus.

In the present specification, the term "optional" or "optionally" means that the event or case described subsequently may or may not occur, and the description includes the case where the event occurs and the case where the event does not occur.

Phrases such as "some specific/preferred embodiments", "other specific/preferred embodiments", and "embodiments" referred to in the present specification mean that particular elements (for example, features, structures, properties and/or characteristics) described in relation to this embodiment are included in at least one of the embodiments described herein, and may or may not exist in other embodiments. Additionally, it should be appreciated that the elements may be combined in any suitable manner into various embodiments.

Before the present disclosure is further described, it shall be understood that the present disclosure is not limited to the particular embodiments described herein. It should also be understood that the terms used herein are intended only to describe rather than limit the particular embodiments.

The term "pharmaceutically acceptable salt" refers to salts of the compounds of the present disclosure that are substantially non-toxic to an organism. Pharmaceutically acceptable salts generally include (but are not limited to) salts formed by reacting the compounds of the present disclosure with a pharmaceutically acceptable inorganic/organic acid or inorganic/organic base, and such salts are also referred to as acid addition salts or base addition salts. Common inorganic acids include (but are not limited to) hydrochloric acid, hydrobromic acid, sulfuric acid, phosphoric acid, and the like. Common organic acids include (but are not limited to) trifluoroacetic acid, citric acid, maleic acid, fumaric acid, succinic acid, tartaric acid, lactic acid, pyruvic acid, oxalic acid, formic acid, acetic acid, benzoic acid, methanesulfonic acid, benzenesulfonic acid, p-toluenesulfonic acid, and the like. Common inorganic bases include (but are not limited to) sodium hydroxide, potassium hydroxide, calcium hydroxide, barium hydroxide, and the like. Common organic bases include (but are not limited to) diethylamine, triethylamine, ethambutol, and the like.

The term "pharmaceutically acceptable carrier" refers to an excipient administered together with the aforesaid nucleic acid lipid nanoparticle composition or the aforesaid pharmaceutical preparation, which is suitable, within the scope of reasonable medical judgment, for contact with the tissue of human beings and/or other animals without undue toxicity, irritation, allergic response or other problems or complications commensurate with a reasonable benefit/risk ratio. Pharmaceutically acceptable carriers usable in the present disclosure include, but are not limited to, a) diluents; b) lubricants; c) binders; d) disintegrants; e) absorbents, colorants, flavoring agents, and/or sweetening agents; f) emulsifiers or dispersants; and/or g) substances that enhance absorption of the compounds.

The term "independently" means that at least two groups (or ring systems) with the same or similar range in the structure may have the same or different meanings under particular circumstances. For example, if the substituent X and the substituent Y are each independently hydrogen, halogen, hydroxyl, cyano, alkyl, or aryl, when the substituent X is hydrogen, the substituent Y may either be hydrogen or be halogen, hydroxyl, cyano, alkyl, or aryl; similarly, when the substituent Y is hydrogen, the substituent X may either be hydrogen or be halogen, hydroxyl, cyano, alkyl, or aryl.

The term "alkyl" refers to monovalent, linear or branched, saturated aliphatic hydrocarbyl. For example, "C₁₋₈ alkyl" refers to monovalent, linear or branched, saturated aliphatic hydrocarbyl including 1 to 8 carbon atoms, *e.g*., alkyl may be methyl, ethyl, n-propyl, isopropyl, n-butyl, sec-butyl, isobutyl, or tert-butyl.

The term "alkylene" refers to divalent, linear or branched, saturated aliphatic hydrocarbyl, in which the two groups (or fragments) it links may be linked either to the same carbon atom or to different carbon atoms. For example, the term "C₁₋₈ alkylene" used herein refers to alkylene having 1 to 8 carbon atoms (such as methylene, 1,1-ethylene, 1,2-ethylene, 1,2-propylene, or 1,3-butylene).

The term "alkenyl" refers to linear or branched, unsaturated aliphatic hydrocarbyl consisting of carbon atoms and hydrogen atoms and having at least one double bond. For example, "C₂₋₃₀ alkenyl" refers to monovalent, linear or branched, hydrocarbyl containing 2 to 30 carbon atoms and having at least one carbon-carbon double bond. Non-limiting examples of alkenyl include, but are not limited to, vinyl, 1-propenyl, 2-propenyl, 1-butenyl, isobutenyl, 1,3-butadienyl, and the like.

The term "branched alkyl", *i.e.* branched chain-containing alkyl, refers to alkyl that is attached to the parent molecule and carries at least two branched structures itself, for example,

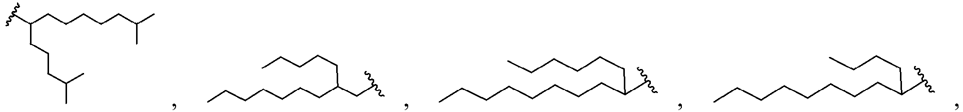

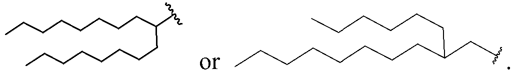

The term "branched alkenyl", *i.e.* branched chain-containing alkenyl, refers to alkenyl that is attached to the parent molecule and carries at least two branched structures itself, for example,

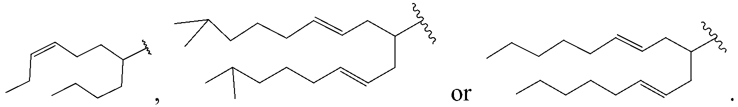

The term "halogen" refers to fluorine (F), chlorine (Cl), bromine (Br) and iodine (I).

In order to render the purposes and technical solutions of the present disclosure clearer, the embodiments of the present disclosure will be described in detail below with reference to the examples. A person skilled in the art will appreciate, however, that the following examples are intended only to illustrate the present disclosure and shall not be considered to limit the scope of the present disclosure.

The reagents or instruments used in the examples are all commercially-available conventional products. Where the specific conditions are not specified, conventional conditions or those recommended by the manufacturers are followed. The term "room temperature" used herein refers to 20°C±5°C. When used to modify a value or a numerical range, the term "about" used herein is meant to include this value or numerical range and an error range of this value or numerical range acceptable to a person skilled in the art, for example, the error range is ±10%, ±5%, ±4%, ±3%, ±2%, ±1%, or ±0.5%.

In the following examples, all the experimental methods employed are conventional methods, unless otherwise specified; and all the reagents and materials are commercially available, unless otherwise specified.

In the following examples, all the solvents and chemicals used are analytically or chemically pure; all the solvents have been re-distilled before use; and all the anhydrous solvents are treated according to standard methods or literature methods.

The abbreviations used herein have the following meanings:

| **Abbrev.** | **Meaning** | **Abbrev.** | **Meaning** |
|---|---|---|---|
| Mg | Magnesium | TMSCl | Trimethylchlorosilane |
| THF | Tetrahydrofuran | EtOH | Ethanol |
| KOH | Potassium hydroxide | DCM | Dichloromethane |
| DIEA | N,N-Diisopropylethylamine | CDI | N,N'-Carbonyldiimidazole |
| MgSO₄ | Magnesium sulfate | TEA | Triethylamine |
| DMAP | 4-Dimethylaminopyridine | MeOH | Methanol |
| Pd/C | Palladium on carbon | EDCI | 1-Ethyl-(3-dimethylaminopropyl)carbodiimide |

¹H NMR spectroscopy is conducted using a Bruker superconducting nuclear magnetic resonance spectrometer (model: AVACE III HD 400 MHz).

Flash silica gel chromatography is conducted using Biotage flash silica gel chromatograph.

### Example 1: Preparation Method for Compound 1

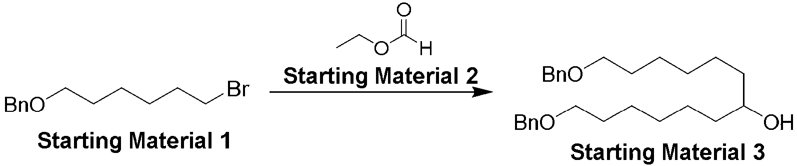

A mixture of **starting material 1** (5 g, 18.44 mmol, 1 *eq*) and THF (20 mL) was added to a mixture of Mg (672.17 mg, 27.66 mmol, 1.5 *eq*) and TMSCl (200.30 mg, 1.84 mmol, 234.00 µL, 0.1 *eq*) in THF (4 mL), and the mixture was stirred at 20°C for 1 hour. A mixture of **starting material 2** (682.89 mg, 9.22 mmol, 741.47 µL, 0.5 *eq*) and THF (10 mL) was added to the reaction mixture, and the reaction mixture was stirred at 20°C for 1 hour. The reaction mixture was poured into a 10% aqueous sulfuric acid solution (30 mL) under ice cooling, to which ethyl acetate (60 mL) was added. The organic layer was separated and dried over anhydrous magnesium sulfate, and then evaporated under reduced pressure to remove the solvent. THF (20 mL), EtOH (10 mL) and KOH (10 M, 5 mL, 2.71 *eq*) were added to the resulting residue, and the mixture was stirred at 40°C for 1 hour. Ethyl acetate (60 mL) and water (50 mL) were added to the reaction mixture. The organic layer was separated, then dried over anhydrous magnesium sulfate, and evaporated under reduced pressure to remove the solvent. The residue was purified by flash silica gel chromatography to give **starting material 3** (1.37 g) as a white solid.

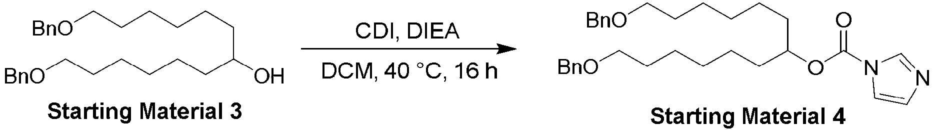

The **starting material 3** (200 mg, 484.73 µmol, 1 *eq*) was dissolved into DCM (5 mL), and DIEA (313.24 mg, 2.42 mmol, 422.15 µL, 5 *eq*) and CDI (235.79 mg, 1.45 mmol, 3 *eq*) were added thereto. The mixture was stirred at 50°C for 16 hours. The reaction mixture was diluted with 20 mL of H₂O and extracted with DCM (40 mL, 20 mL * 2). The combined organic layer was washed with brine (30 mL), dried over anhydrous MgSO₄, filtered and concentrated under reduced pressure to give **starting material 4** as a yellow gel-like product.

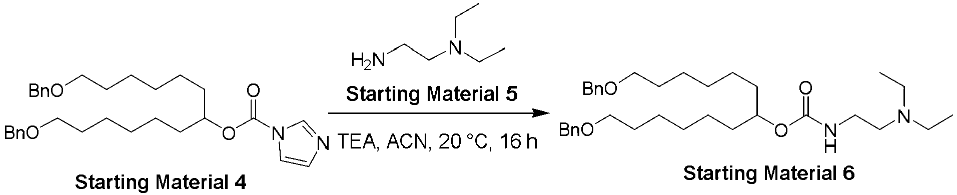

To a solution of **starting material 4** in acetonitrile (5 mL) were added TEA (419.40 mg, 4.14 mmol, 576.88 µL, 10 *eq),* DMAP (50.63 mg, 414.47 µmol, 1 *eq*) and **starting material 5** (481.63 mg, 4.14 mmol, 582.38 µL, 10 *eq).* The mixture was stirred at 50°C for 16 hours. The residue was purified by flash silica gel chromatography to give **starting material 6** as a colorless oily product.

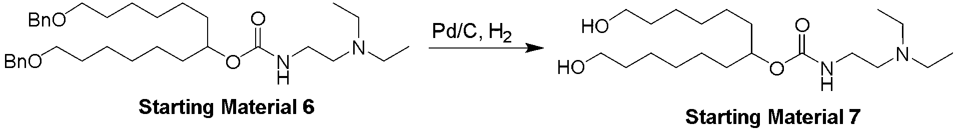

To a solution of **starting material 6** (190 mg, 308.22 µmol, 1 *eq)* in MeOH (5 mL) was added Pd/C (32.80 mg, 30.82 µmol, 10% purity, 0.1 *eq*)*.* The suspension was degassed under vacuum and purged several times with hydrogen. The mixture was stirred under hydrogen (50 psi) at 50°C for 16 hours. The mixture was stirred at 50°C for 16 hours. The mixture was concentrated under reduced pressure to give **starting material 7** as a colorless oily product.

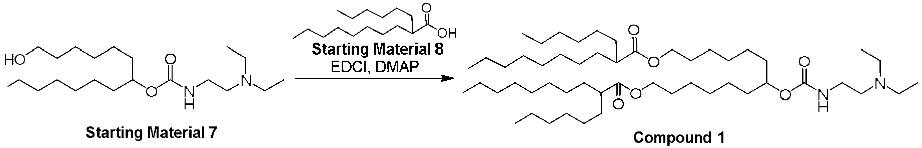

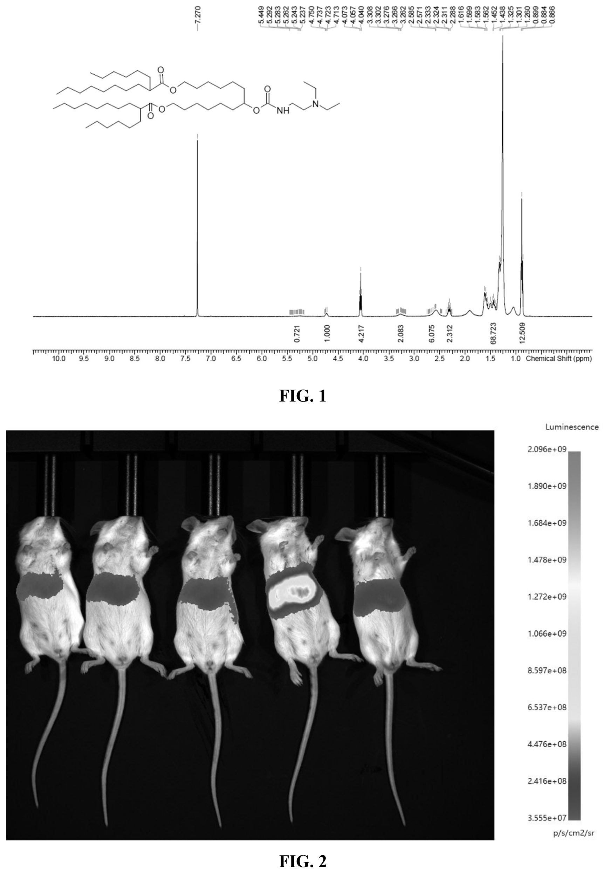

The **starting material 7** (100 mg, 266.98 µmol, 1 *eq*) and 2-hexyldecanoic acid (205.38 mg, 800.95 µmol, 3 *eq*) were dissolved into DCM (3 mL), and EDCI (102.36 mg, 533.96 µmol, *2 eq*) and DMAP (3.26 mg, 26.70 µmol, 0.1 *eq*) were added thereto. The reaction mixture was stirred at 15°C for 18 hours. The reaction mixture was diluted with H₂O (60 mL) and extracted with DCM (100 mL, 50 mL * 2). The combined organic layer was washed with brine (100 mL), dried over anhydrous MgSO₄, filtered and concentrated under reduced pressure to give a residue. The residue was purified by flash silica gel chromatography to give **compound 1** as a colorless liquid. The ¹H NMR spectral data for compound 1 was as shown in FIG. 1.

### Example 2: Preparation Method for Compound 14

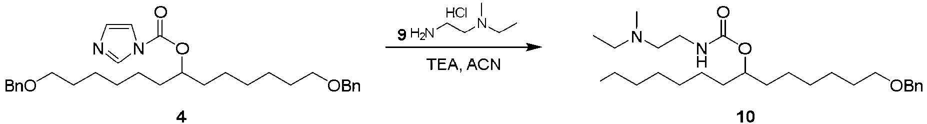

At 25°C, TEA was added to a solution of [7-benzyloxy-1-(6-benzyloxyhexyl)heptyl]imidazole-1-carboxylate and N'-ethyl-N'-methyl-ethane-1,2-diamine in ACN. After addition, the mixture was warmed at 50°C and stirred at 50°C for 16 hours.

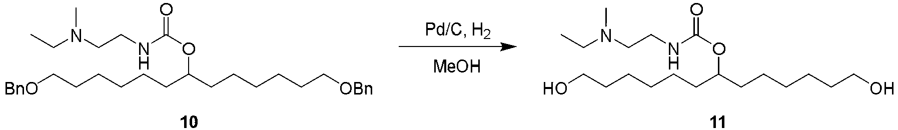

In an Ar₂ atmosphere at 25°C, Pd/C was added to a solution of [7-benzyloxy-1-(6-benzyloxyhexyl)heptyl]-N-[2-[ethyl(methyl)amino]ethyl]carbamate in MeOH. After addition, the mixture was warmed at 50°C and stirred at 50°C for 16 hours.

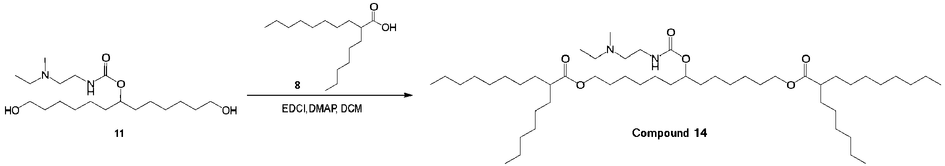

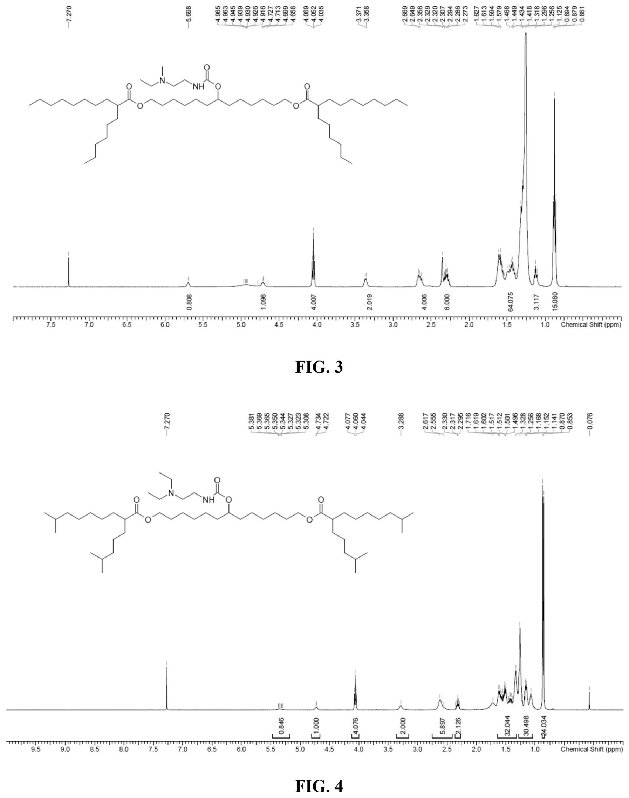

At 25°C, DMAP was added to a solution of [7-hydroxy-1-(6-hydroxyhexyl)heptyl]-N-[2-[ethyl(methyl)amino]ethyl]carbamate and 2-hexyldecanoic acid in DCM. The mixture was cooled at 0°C, and EDCI was added at 0°C in an N₂ atmosphere. After addition, the mixture was warmed at 25°C and stirred at 25°C for 16 hours. The reaction mixture was worked up to give compound 14 (yield: 95%). The ¹H NMR spectral data for compound 14 was as shown in FIG. 3.

### Example 3: Preparation Method for Compound 15

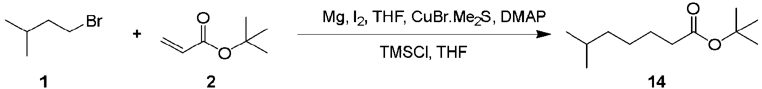

Mg and I₂ were charged into a 250-mL round bottom flask, THF was added thereto, and 1-bromo-3-methylbutane was added dropwise. The reaction mixture was stirred at 20°C for 2 hours under N₂. Subsequently, DMAP and cuprous bromide-dimethyl sulfide were added rapidly. The flask was cooled at -70°C, and tert-butyl prop-2-enoate, TMSCl and THF were added dropwise. The mixture was stirred at 20°C for 12 hours under N₂.

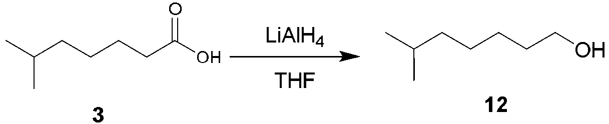

A solution of LiAlH₄ in THF was cooled at 0°C under N₂, and then a solution of 6-methylheptanoic acid in THF was added at 0°C. The mixture was stirred at 25°C for 2 hours under N₂.

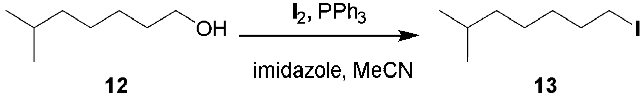

A solution of PPh₃ and imidazole in MeCN was cooled at 0°C under N₂, and then a solution of 6-methylheptan-1-ol in MeCN was added at 0°C. The mixture was stirred at 0°C for 15 minutes. Subsequently, I₂ was added in batches. The mixture was stirred at 20°C for 1.5 hours under N₂.

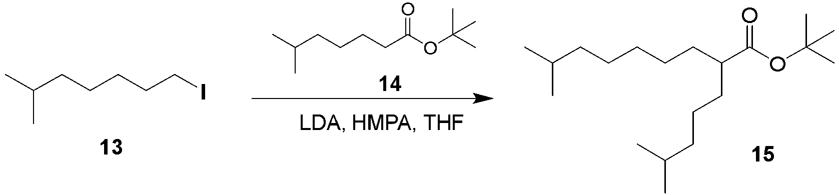

Under N₂, a solution of N-isopropylpropan-2-amine in THF was cooled at -70°C, and then n-BuLi was added. Under N₂, the mixture was stirred at -70°C for 0.5 hours. The reaction mixture was warmed at 0°C. A solution of tert-butyl 6-methylheptanoate in THF was cooled at 0°C, and then a LDA solution was added. The mixture was stirred at 0°C for 30 minutes. Under N₂, 1-iodo-6-methylheptane and HMPA were added dropwise at 0°C. The mixture was stirred under N₂ at 20°C for 12 hours. The reaction mixture was worked up and purified to give tert-butyl 2-isohexyl-8-methylnonanoate as a light yellow oily product.

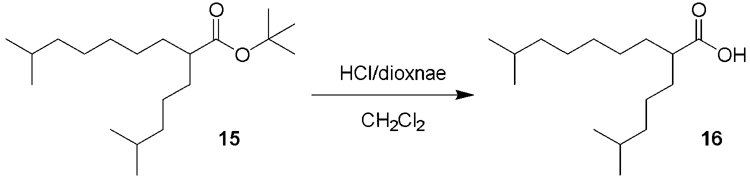

To a solution of tert-butyl 2-isohexyl-8-methylnonanoate in CH₂Cl₂ (2 ml) was added HCl/dioxane. The mixture was stirred at 20°C for 24 hours.

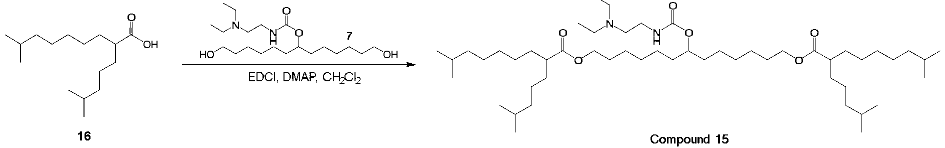

To a solution of 2-isohexyl-8-methylnonanoic acid and [7-hydroxy-1-(6-hydroxyhexyl)heptyl]-N-[2-(diethylamino)ethyl]carbamate in CH₂Cl₂ (20 mL), DMAP was added and stirred at 20°C for 0.5 hours. Subsequently, EDCI was added. The reaction mixture was stirred at 20°C for 12 hours. The ¹H NMR spectral data for compound 15 was as shown in FIG. 4.

### Example 4: Preparation and Characterization of Lipid Nanoparticles

### 1. Preparation of lipid nanoparticles (LNPs)

The preparation of lipid nanoparticles involved mixing the above-mentioned ionizable lipid, helper lipid, structural lipid, polymer-conjugated lipid and the like at a certain molar ratio, and dissolving the mixture into anhydrous ethanol, so that the total lipid phase concentration was 0.5 to 20 mg/mL. Nucleic acids were dissolved into a buffer solution having a pH of less than 7 (*e.g.,* 3 to 6.5), and rapidly mixed with a solution of lipids in ethanol to achieve the preparation purpose.

In this example, the ionizable lipid (MC3, compound 1, compound 14 or compound 15), the helper lipid (DSPC or DOPE), the structural lipid (cholesterol) and the polymer-conjugated lipid (DMG-PEG2000) were dissolved into anhydrous ethanol at the molar ratio shown in Table 1 to formulate a solution with a total lipid phase concentration of 1 mg/mL.

Luc-eGFP mRNA at the corresponding concentration was dissolved in 50 mM citric acid buffer with an aqueous buffer solution of pH4.0. The mass ratio of mRNA to the total lipids was 1:30.

LNPs were prepared using the formulation-screening chip from **Micro & Nano** at a total flow rate of 12 ml/min and a flow rate ratio of 1:3 between the lipid phase and the aqueous phase. The lipid nanoparticles were collected and dialyzed with PBS overnight at 4°C to remove ethanol and acidic buffer salts. The dialysate volume was 400 times or more the sample volume. The dialyzed sample was filtered through a 0.2-µm filter membrane. Lipid nanoparticles could also be prepared by other methods, such as injection mixing, in addition to using microfluidic chips. After being dialyzed and filtered, the sample was concentrated using a 10 kDa cellulose ultrafiltration membrane. The centrifugation parameter was 1000 g to 2000 g, and the centrifugation time was 30 min to 1 hour. The centrifugation temperature was 4°C.

### 2. Determination of particle size: The particle size and polydispersity index (PDI) of the prepared lipid nanoparticles were measured using a Malvern particle size analyzer.

### 3. Determination of encapsulation efficiency of mRNAs:

For mRNA-encapsulated lipid nanoparticles, the assay specified in the Quant-iT^{™} RiboGreen^{®} RNA kit could be employed. The specific operations were as follows: The sample was diluted 10 to 20 folds with a TE buffer solution (10 mM Tris-HCl, 1 mM EDTA, pH 7.5). An equal volume of demulsifier (2% Triton X-100) was added to the sample to be tested and then the solution was diluted 10 to 20 folds again. The RiboGreen^{®} reagent was diluted 100 folds in the TE buffer solution, and 100 µl of the diluted solution was added to a 96-well plate. 100 µl of the sample to be tested was added to the corresponding 96-well plate. Meanwhile, a standard curve was plotted using the mRNA standard. The fluorescence value at 520 nm excited by 480 nm excitation light was read using a microplate reader. The concentrations of the encapsulated and free mRNAs were calculated based on the standard curve. The encapsulation efficiency was calculated by the ratio of the concentration of the encapsulated mRNAs to the total mRNA concentration.

The constituents and the characterization data of the lipid nanoparticles were as shown in Table 1.

**Table 1: Particle Sizes and Nucleic Acid Encapsulation Efficiencies of Lipid Nanoparticles**

| Experimental Conditions | Composition and Molar Ratio of LNP | Particle Size (nm) | PDI | Encapsulation Efficiency (%) |
|---|---|---|---|---|
| 1 | MC3:DSPC:cholesterol:DMG-PEG-2000 | 78.53 | 0.147 | 95.54 |
| | 50:10:38.5:1.5 | | | |
| 2 | Compound 1:DSPC:cholesterol:DMG-PEG-2000 | 84.00 | 0.264 | 93.11 |
| | 50:10:38.5:1.5 | | | |
| 3 | Compound 1:DSPC:cholesterol:DMG-PEG-2000 | 60.45 | 0.240 | 88.91 |
| | 50:10:37:3 | | | |
| 4 | Compound 1:DOPE:cholesterol:DMG-PEG-2000 | 77.27 | 0.249 | 91.56 |
| | 50:10:38.5:1.5 | | | |
| 5 | Compound 1:DOPE:cholesterol:DMG-PEG-2000 | 64.99 | 0.335 | 88.49 |
| | 50:10:37:3 | | | |
| 6 | Compound 14:DOPE:cholesterol:DMG-PEG-2000 | 110.3 | 0.314 | 94.20 |
| | 50:10:38.5:1.5 | | | |
| 7 | Compound 15:DOPE:cholesterol:DMG-PEG-2000 | 77.81 | 0.181 | 97.38 |
| | 50:10:38.5:1.5 | | | |

### Example 5: Experiment on In Vivo Delivery of Luciferase mRNA in Animals

The nucleic acid delivery efficiency of lipid nanoparticles was characterized by observing the fluorescence expression in mice. The Luc-eGFP mRNA-encapsulated lipid nanoparticles prepared in Example 4 were injected at a certain dose into mice via the tail vein. Female C57/BL6 mice weighing 18 to 22 g were randomly grouped, with two mice in each group. After acclimation, Luc-eGFP mRNA-encapsulated LNPs (in a sterile PBS solution) were injected via the tail vein at a dose of 0.5 mg/kg (0.5 mg here referring to the dose of mRNA). In order to observe the expression of luciferase, the luciferase substrate was dissolved into a sterile PBS solution and prepared into a solution at a concentration of 30 mg/ml.

Six hours after injection of LNPs, 150 ul of luciferase substrate solution was injected intraperitoneally into each mouse. The mice were left to stand for 5 min, and then anesthetized for 3 min in a carbon dioxide box. After anesthesia, the mice were put in a small animal *in vivo* imager for imaging.

**Table 2: Detection of Chemiluminescence Intensity from Luc-eGFP mRNA Delivered by Lipid Nanoparticles**

| Experimental Conditions | Composition and Molar Ratio of LNP | Luminous Intensity 6h (p/s/cm²/sr) |
|---|---|---|
| 1 | MC3:DSPC:cholesterol:DMG-PEG-2000 | 1.35E+8 |
| | 50:10:38.5:1.5 | |
| 2 | Compound 1:DSPC:cholesterol:DMG-PEG-2000 | 2.26E+8 |
| | 50:10:38.5:1.5 | |
| 3 | Compound 1:DSPC:cholesterol:DMG-PEG-2000 | 2.35E+8 |
| | 50:10:37:3 | |
| 4 | Compound 1:DOPE:cholesterol:DMG-PEG-2000 | 2.09E+9 |
| | 50:10:38.5:1.5 | |
| 5 | Compound 1:DOPE:cholesterol:DMG-PEG-2000 | 4.16 E+8 |
| | 50:10:37:3 | |
| 6 | Compound 14:DOPE:cholesterol:DMG-PEG-2000 | 2.6 E+8 |
| | 50:10:38.5:1.5 | |
| 7 | Compound 15:DOPE:cholesterol:DMG-PEG-2000 | 1.10 E+9 |
| | 50:10:38.5:1.5 | |

The mice in FIG. 2 were mice subjected to experimental conditions 1, 2, 3, 4, and 5 from left to right.

As shown in Table 2 and FIG. 2, compared with the commonly used ionizable lipids MC3, the lipid nanoparticles formed from the compounds of the present disclosure could significantly improve the delivery efficiency of nucleic acids in mice and exhibited good liver targeting and a high transfection potential in liver, confirming their feasibility of *in vivo* applications.

## Claims

1. A compound represented by formula (1) or a pharmaceutically acceptable salt thereof: 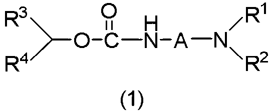 wherein
R¹ and R² are independently C₁₋₈ alkyl, and said alkyl is optionally substituted with one or more of OH, NH₂ or halogen;
A is C₁₋₈ alkylene;
R³ and R⁴ are independently -R⁶, -G³-O-C(=O)-R⁵, -G³-C(=O)-O-R⁵, -G³-NH-C(=O)-O-R⁵, -G³-NH-O-C(=O)-R⁵, -G³-NH-C(=O)-R⁵ or -G³-O-C(=O)-NH-R⁵;
G³ is independently C₂₋₁₀ alkylene;
R⁵ is independently C₂₋₃₀ branched alkyl or C₂₋₃₀ branched alkenyl, and said branched alkyl or branched alkenyl is optionally substituted with one or more of -O-C₁₋₃₀ alkyl, -C(=O)-O-C₁₋₃₀ alkyl, -O-C(=O)-C₁₋₃₀ alkyl, -O-C₂₋₃₀ alkenyl, -C(=O)-O-C₂₋₃₀ alkenyl or -O-C(=O)-C₂₋₃₀ alkenyl; and
R⁶ is C₂₋₃₀ alkenyl.

2. The compound or the pharmaceutically acceptable salt thereof according to claim 1, wherein
R¹ and R² are independently C₁₋₆ alkyl, and said alkyl is optionally substituted with one or more of OH, NH₂ or halogen; or
R¹ and R² are independently C₁₋₄ alkyl, and said alkyl is optionally substituted with one or more of OH, NH₂ or halogen; or
R¹ and R² are independently -CH₃, -CH₂CH₃, -CH₂CH₂OH or -CH₂CH₂CH₃; or
R¹ and R² are independently -CH₂CH₃ or -CH₂CH₂OH.

3. The compound or the pharmaceutically acceptable salt thereof according to claim 1 or 2, wherein
A is C₁₋₆ alkylene; or
A is C₁₋₄ alkylene; or
A is -CH₂-, -CH₂CH₂- or -CH₂CH₂CH₂-; or
A is -CH₂CH₂-.

4. The compound or the pharmaceutically acceptable salt thereof according to any one of claims 1 to 3, wherein
R³ and R⁴ are independently -R⁶, -G³-O-C(=O)-R⁵, -G³-C(=O)-O-R⁵, -G³-NH-C(=O)-O-R⁵ or -G³-NH-O-C(=O)-R⁵; or
R³ and R⁴ are independently -R⁶, -G³-O-C(=O)-R⁵ or -G³-C(=O)-O-R⁵; or
R³ and R⁴ are independently -R⁶ or -G³-O-C(=O)-R⁵; or
R³ and R⁴ are independently -G³-O-C(=O)-R⁵. or
R³ and R⁴ are independently 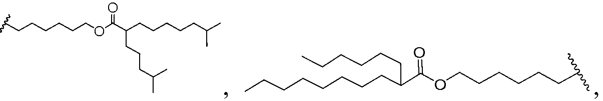 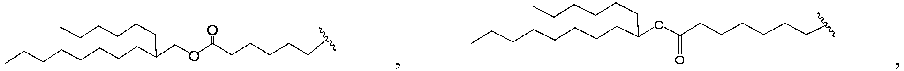 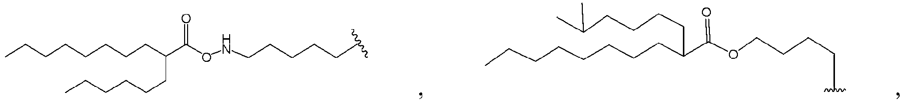 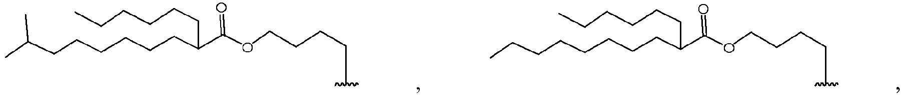 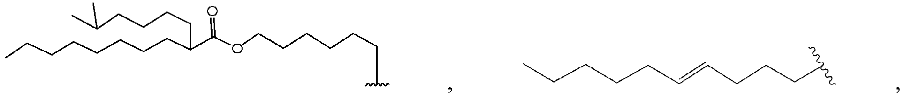 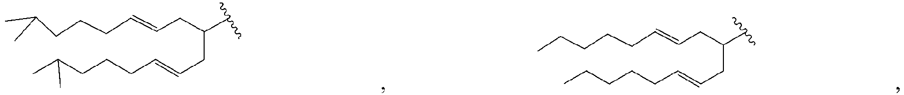 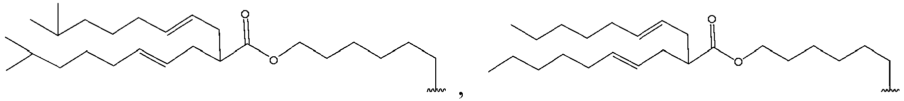 or 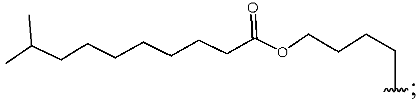 or
R³ and R⁴ are independently 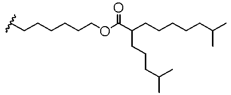 or

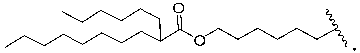

5. The compound or the pharmaceutically acceptable salt thereof according to any one of claims 1 to 4, wherein
G³ is independently C₂₋₈ alkylene; or
G³ is independently C₂₋₆ alkylene; or
G³ is independently 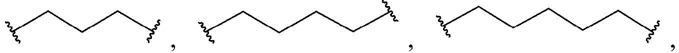 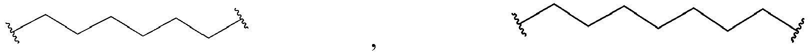 or 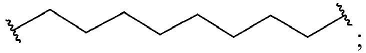 or
G³ is independently 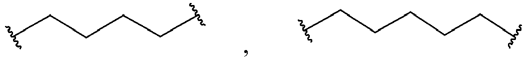 or

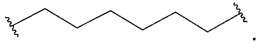

6. The compound or the pharmaceutically acceptable salt thereof according to any one of claims 1 to 5, wherein
R⁵ is C₂₋₂₄ branched alkyl or C₂₋₂₄ branched alkenyl, and said branched alkyl or branched alkenyl is optionally substituted with one or more of -O-C₁₋₂₄ alkyl, -C(=O)-O-C₁₋₂₄ alkyl, -O-C(=O)-C₁₋₂₄ alkyl, -O-C₂₋₂₄ alkenyl, -C(=O)-O-C₂₋₂₄ alkenyl or -O-C(=O)-C₂₋₂₄ alkenyl; or
R⁵ is C₈₋₂₄ branched alkyl or C₈₋₂₄ branched alkenyl, and said branched alkyl or branched alkenyl is optionally substituted with one or more of -O-C₁₋₂₄ alkyl, -C(=O)-O-C₁₋₂₄ alkyl, -O-C(=O)-C₁₋₂₄ alkyl, -O-C₂₋₂₄ alkenyl, -C(=O)-O-C₂₋₂₄ alkenyl or -O-C(=O)-C₂₋₂₄ alkenyl; or
R⁵ is 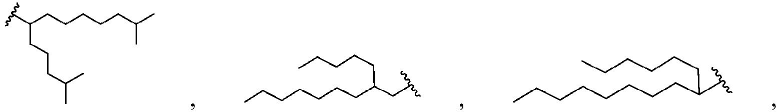 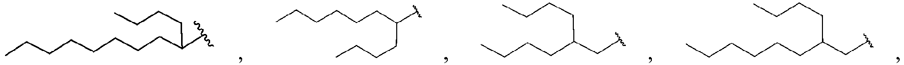 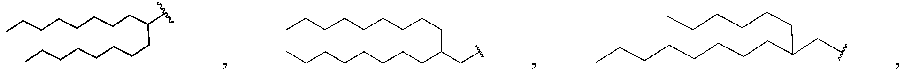 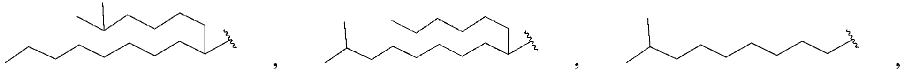 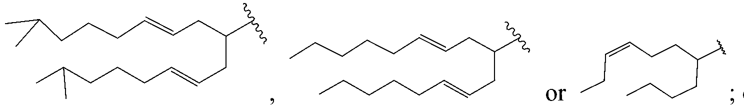 or
R⁵ is

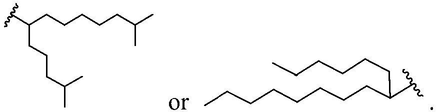

7. A compound or a pharmaceutically acceptable salt thereof, wherein the compound is selected from the following compounds:
| | Structure of Compound |
|---|---|
| 1 | 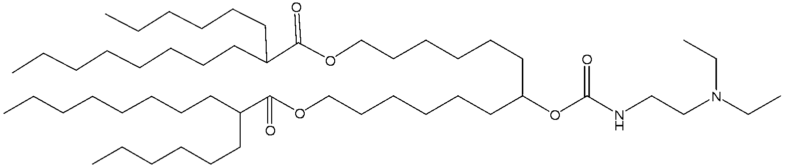 |
| 2 | 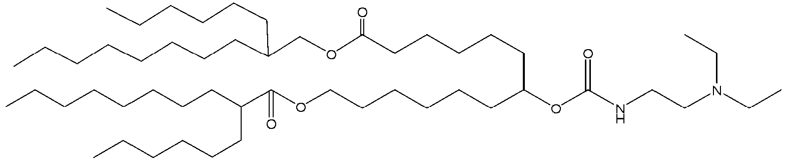 |
| 3 | 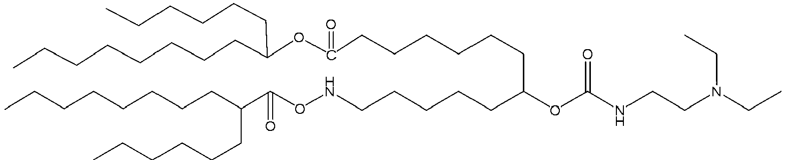 |
| 4 | 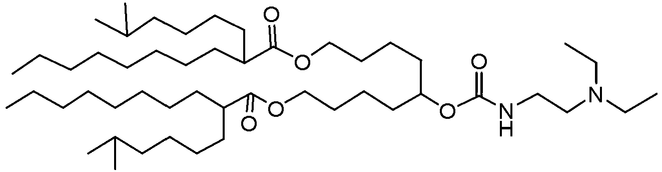 |
| 5 | 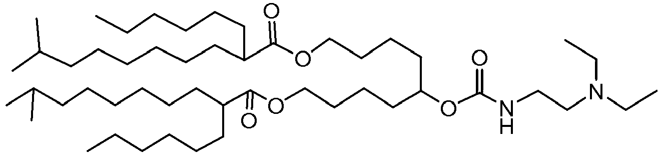 |
| 6 | 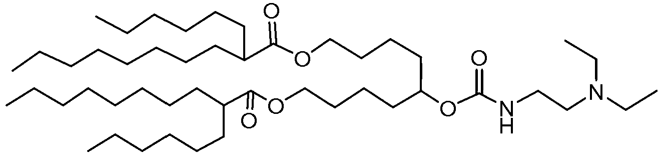 |
| 7 | 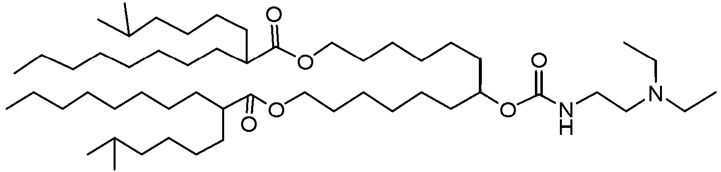 |
| 8 | 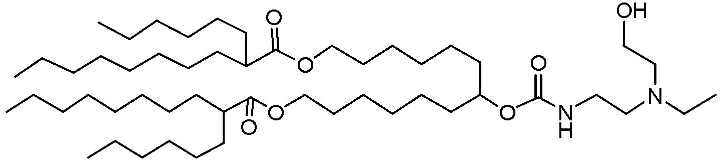 |
| 9 | 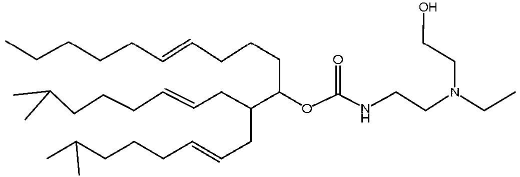 |
| 10 | 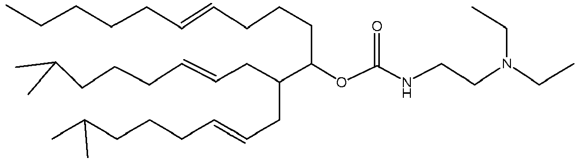 |
| 11 | 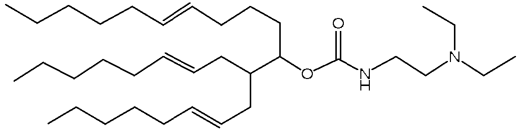 |
| 12 | 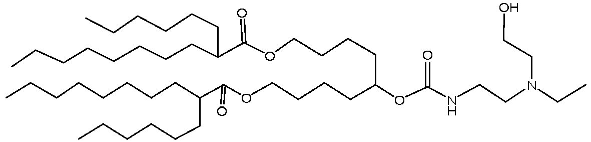 |
| 13 | 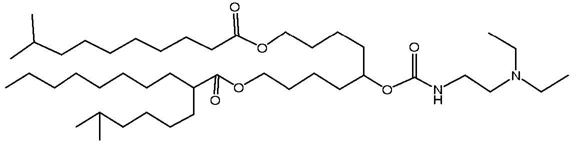 |
| 14 | 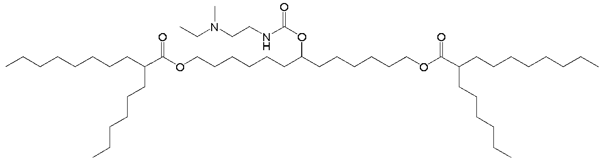 |
| 15 | 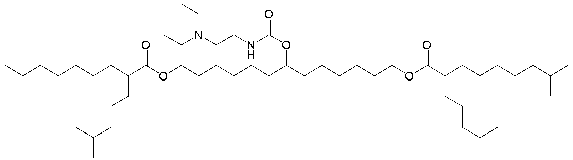 |

8. A lipid carrier, comprising the compound or the pharmaceutically acceptable salt thereof according to any one of claims 1 to 7 as an ionizable lipid.

9. The lipid carrier according to claim 8, further comprising a helper lipid, a structural lipid, and a polymer-conjugated lipid.

10. A nucleic acid lipid nanoparticle composition, comprising the compound or the pharmaceutically acceptable salt thereof according to any one of claims 1 to 7 or the lipid carrier according to claim 8 or 9, and a nucleic acid.

11. The nucleic acid lipid nanoparticle composition according to claim 10, wherein the nucleic acid is at least one selected from DNA, RNA, a complex containing DNA or RNA, a modified DNA, a modified RNA, and a modified complex containing DNA or RNA.

12. A pharmaceutical preparation, comprising the compound or the pharmaceutically acceptable salt thereof according to any one of claims 1 to 7, the lipid carrier according to claim 8 or 9, or the nucleic acid lipid nanoparticle composition according to claim 10 or 11, and a pharmaceutically acceptable carrier.

13. Use of the compound or the pharmaceutically acceptable salt thereof according to any one of claims 1 to 7, the lipid carrier according to claim 8 or 9, the nucleic acid lipid nanoparticle composition according to claim 10 or 11, or the pharmaceutical preparation according to claim 12 in preparation of a nucleic acid drug or a gene vaccine.
